# EUROPÄISCHE PATENTSCHRIFT

(11) **EP 3 494 922 B1**
(45) Veröffentlichungstag und Bekanntmachung des Hinweises auf die Patenterteilung: **07.06.2023**
(21) Anmeldenummer: 18198191.1
(22) Anmeldetag: 09.11.2012
(51) Int. Cl.: A61C 13/00, A61C 13/01, A61C 13/20

(54) **VERFAHREN ZUR HERSTELLUNG VON TEIL- ODER TOTALPROTHESEN**
METHOD FOR THE PREPARATION OF PARTIAL OR TOTAL PROSTHESES
PROCÉDÉ DE FABRICATION DE PROTHÈSES PARTIELLES OU TOTALES

(30) Priorität: 11.11.2011 DE 102011118320
(43) Veröffentlichungstag der Anmeldung: 12.06.2019
(62) Teilanmeldung aus: 12788440.1
(73) Patentinhaber: Merz Dental GmbH, 24321 Lütjenburg (DE)
(72) Erfinder: Klingenburg, Friedhelm, 24321 Lütjenburg (DE); Frölich, Carola, 89073 Ulm (DE)
(74) Vertreter: Fish & Richardson P.C.

(56) Entgegenhaltungen:
- WO-A1-2010/057584
- DE-A1- 4 025 728
- US-A- 3 846 911

## Beschreibung

Die Erfindung betrifft einen Fräsblock, ein Verfahren zur Herstellung von Teil- oder Totalprothesen, sowie ein komplettes Fräsblocksystem bestehend aus einem ersten Fräsblock für das Obergebiss und einem zweiten Fräsblock für das Untergebiss und ein Verfahren zur Bereitstellung einer Teil- oder Totalprothese.

Aus der EP 0 501 983 B1 ist bereits ein Verfahren zur Herstellung einer Totalprothese mit einer Ober- und/oder Unterkieferbasis mit Hilfe einer Fräseinrichtung bekannt, wobei die jeweilige Basis aus einem Kunststoffblock ausgefräst wird und die Kontur oder Topographie kennzeichnenden Daten der Mundhöhle verwendet werden. Dies darin beschriebene Verfahren verwendet ferner nach Erstellen der angepassten Basis an die Mundhöhle den weiteren Verfahrensschritt, notwendige Zähne in Wachs auf die Ober- und/oder Unterkieferbasis aufzustellen, wobei die Fertigstellung der Prothese durch Verbinden der Zähne mit der Basis mit Hilfe eines Kaltpolymerisates durchgeführt wird. Daran anschließend erfolgt das sogenannte Reokkludieren, damit dem jeweiligen Patient entsprechend seiner Kaubewegungen ein optimaler Biss vermittelt wird.

Dieses bekannte Verfahren beinhaltet somit den Nachteil, dass nach Fertigstellung der Ober- und/oder Unterkieferbasis ein individuelles Anpassen an den Patienten durch entsprechendes Aufstellen der Zähne in Wachs erforderlich wird, was wiederum den gesamten zahntechnischen und zahnärztlichen Behandlungsablauf zeit- und kostenintensiv macht.

Ferner wird in diesem Zusammenhang auf die DE4025728 A1 und die WO 2010/057584 A1 verwiesen.

Aufgabe der folgenden Erfindung ist es somit, ein Verfahren zur Herstellung von Teil- oder Totalprothesen, welche die Nachteile des Standes der Technik vermeiden. Ferner ist es Aufgabe der folgenden Erfindung, die Arbeitsschritte zur individuellen Anpassung an den Patienten zu verringern.

Die vorliegende Erfindung wird durch den unabhängigen Anspruch 1 definiert. Die abhängigen Ansprüche zeigen andere Ausführungsformen der Erfindung.

Erfindungsgemäß wird zur Lösung der Aufgaben vorgeschlagen, dass ein Fräsblock zur Herstellung von Teil- oder Totalprothesen bereitgestellt wird, bei dem entsprechend der Kieferform eine zu bearbeitende Prothesenbasis mit künstlich geformten Zähnen eines vollständigen Gebisses vorgesehen ist, wobei die Zähne für die Okklusionsstellung aufgestellt sind. In diesem Sinne ist auch das entsprechende Fräsblocksystem hinsichtlich Obergebiss und Untergebiss unabhängig von der Befestigung im Mund ausgestattet.

Mit dieser anmeldungsgemäßen Maßnahme wird erreicht, dass lediglich die Prothesenbasis gefräst bzw. geformt werden muss, und bei korrekter Fräsung entsprechend vorliegender CAD/CAM-Daten der Mundhöhle des Patienten aufgrund bereits auf der Prothesenbasis verankerter künstlich geformter Zähne keine weitere Anpassung erfolgen muss.

Durch entsprechend optimierte Einpassung und Justierung der Prothesenbasis in die Mundhöhle wird somit nur ein individueller Anpassungsschritt erforderlich sein, um den Patienten optimal zu versorgen.

Aufgrund des anmeldungsgemäßen Vorhandenseins von einem Fräsblock bzw. Fräsblocksystem, bei dem eine entsprechende Kieferform zur bearbeitenden Prothesenbasis mit künstlich geformten Zähnen vorliegt, muss weder eine separate Bissnahme für eine Kieferrelationsbestimmung, noch eine zeit- und kostenintensive Anprobe vorgenommen werden.

Mit der anmeldungsgemäßen Maßnahme ist somit ein nachträgliches Einsetzen in einem Artikulator zur Bestimmung der Positionierung der Zähne nicht erforderlich, da die künstlich geformten Zähne bereits entsprechend gegebener Grunddaten für die Okklusionsstellung vorbereitet sind.

Mit dem anmeldungsgemäßen Verfahren zur Bereitstellung einer Teil- oder Totalprothese ist es somit möglich, dass aufgrund des Vorhandenseins von in Okklusionsstellung vorliegenden Zahnober- und unterreihen in Gebissform mindestens drei unterschiedliche Fräsblocksysteme mit unterschiedlichen Größenverhältnissen in der Gebissform und Größe der Zähne bereitgestellt werden können, um nahezu alle Patienten versorgen zu können. Mit dieser Maßnahme wird ein zeit- und kosteneffizientes Herstellungsverfahren erzielt und dem Zahnarzt und/oder dem Zahntechniker in allen Ländern eine vollständige Versorgung der Bevölkerung ermöglicht.

Im Unterschied zu den bekannten Dentalprothesen bzw. deren Herstellungsverfahren richtet sich der erfindungsgemäße Fräsblock auf eine einstückige Anordnung mit einem ersten Bereich, welcher eine entsprechend der Kieferform bearbeitbare Prothesenbasis aufweist und einem zweiten Bereich mit einer vorbestimmten, bzw. standardisierten Anordnung künstlich geformter Zähne oder Zahnreihen für ein Ober- bzw. Unterkiefergebiss. Damit wird als Vorstufe zur Dentalprothese ein Fräsblock bereitgestellt, der besonders kosten- und zeitsparend lediglich in seinem ersten Bereich, nämlich der Prothesenbasis, zur Anpassung an die Kieferform bearbeitbar, d.h. fräsbar ist. Das erfindungsgemäße Fräsblocksystem umfasst einen ersten Fräsblock für das Obergebiss und einen zweiten Fräsblock für das Untergebiss, d.h. die Vorstufen einer Teil- oder Totalprothese, welche im jeweils zweiten Bereich des ersten und zweiten Fräsblocks eine vorbestimmte bzw. standardisierte und nach gängigen Okklusionsprinzipien aufgestellte Anordnung künstlich geformter Zähne oder Zahnreihen für das Ober- bzw. Unterkiefergebiss aufweist.

Eine aus dem Stand der Technik bekannte Mehrzahl von Vorformlingen mit verschiedenen Farben, Größen- und Formenkombinationen für den Schleimhautbereich und Zahnbereich zur Herstellung von Zahnersatz wird mit vorliegendem Erfindungsgegenstand durch die vorbestimmte bzw. standardisierte Anordnung künstlich geformter, einpolymerisierter Zähne oder Zahnreihen für das Ober- bzw. Unterkiefergebiss erheblich vereinfacht. Vorteilhaft ist anmeldungsgemäß, dass lediglich nur die jeweilige, dem Schleimhautbereich zugeordnete Prothesenbasis zu fräsen ist.

Darüber hinaus handelt es sich bei der vorbestimmten bzw. standardisierten und nach gängigen Okklusionsprinzipien aufgestellten Anordnung künstlich geformter Zähne oder Zahnreihen für das Ober- bzw. Unterkiefergebiss um nicht-individualisierte, vorgefertigte Zahnersatzteile, die keiner weiteren Nachbearbeitung bedürfen.

Die vorbestimmte bzw. standardisierte und nach gängigen Okklusionsprinzipien aufgestellten Anordnung künstlich geformter Zähne oder Zahnreihen für das Ober-bzw. Unterkiefergebiss erfolgt nach einer Auswahl aus vorgefertigten Zahneratzteilen, d.h. künstlich geformter Zähne oder Zahnreihen nach vorgegebenen und bekannten Aufstellsystemen.

Weitere vorteilhafte Weiterbildungen der Erfindung sind Gegenstand der Unteransprüche.

Sind darüber hinaus die künstlich geformten Zähne und die Prothesenbasis einstückig ausgebildet, so kann bei der Herstellung des Fräsblockes nahezu eine Guss- bzw. Pressform verwendet werden, die somit auch jeweils nur ein Material für den Fräsblock verwendet, welches vorteilhaft sowohl ein geringeres Allergiepotential als auch eine höhere Verbundfestigkeit aufweist.

Ist vorteilhafterweise der Fräsblock zweiteilig ausgestaltet, so ist die Möglichkeit gegeben, dass der erste Bereich, der entsprechend der Kieferform bearbeitet wird, standardisiert hergestellt werden kann und mit unterschiedlichen, ebenfalls schon auf einem zweiten Bereich aufgestellten Zähnen, welche unterschiedliche Größen und Gegebenheiten aufweisen, zusammengefügt werden kann. Mit der Weiterbildung, dass die Prothesenbasis flexibel ausgestaltet wird, kann einer vereinfachten Einpassung in die Mundhöhle bzw. Anpassung an die Kieferform des jeweiligen Patienten Rechnung getragen werden.

Als besonders vorteilhafte Materialien für den Fräsblock bzw. für das Fräsblocksystem, d. h. Prothesenbasis und/oder Zähne, bieten sich u. a. sogenannte PMMA-Kunststoffe, ebenso als Thermoplaste, organisch als auch anorganisch gefüllte Kunststoffe und auch allergiearme Kunststoffe an. Zusätzlich ist es ebenfalls vorteilhaft, jedwede Form von Keramiken, insbesondere Glaskeramiken, leuzitverstärkte Keramik, aluminiumverstärkte Keramik, Oxidkeramik, Zirkonoxidkeramik, Infiltrationskeramik, Feldspatkeramiken, Lithium-Disilikat-Keramik, zu verwenden. Denkbar ist es ebenfalls, Materialien zu verwenden, die gesintert werden, die jedoch noch einen weiteren Fertigungsschritt benötigen. Verwendbar ist der Fräsblock bzw. das Fräsblocksystem auch bei kombiniertem Zahnersatz.

Sind die Zähne entsprechend einer Okklusionsstellung bereits ausgerichtet, wird somit nur durch entsprechende Fräsung der Prothesenbasis die vollständige Einpassung in die Mundhöhle des Patienten vorgenommen. Der Patient kann damit sicher gehen, dass bei entsprechender Anpassung in die Mundhöhle bzw. an den Ober- bzw. Unterkiefer eine optimale Okklusionsstellung bereitsteht und somit vorteilhaft eine hinsichtlich des Bissverhaltens uneingeschränkt funktionsfähige Totalprothese vorliegt. Aufgrund des anmeldungsgemäßen Fräsblockes bzw. Fräsblocksystems und dem entsprechenden Verfahren zur Herstellung von Teil- oder Totalprothesen ist es beispielsweise möglich, mindestens drei unterschiedliche Größen bereitzustellen, mit denen nahezu für jeden Patienten entsprechend seiner Gesichtsform und Größe der Mundhöhle eine vollständige Totalprothese bereitgestellt werden kann. Aufgrund der Einfachheit der Anpassung der Totalprothese sind somit die Arbeitsschritte zur Anpassung in die Mundhöhle erheblich reduziert und damit auch kostengünstiger. Je nach Erfahrungswerten kann bei bestimmten Bevölkerungsgruppen das Fräsblocksystem auch nur zwei Arten oder mehr beinhalten.

Hinsichtlich der Vorbereitung und Aufstellung der künstlich geformten Zähne sind bei dem anmeldungsgemäßen Fräsblock, Fräsblocksystem und dem entsprechenden Verfahren alle hierzu möglichen, bislang bekannten Aufstellsysteme einsetzbar, wie z. B. die sogenannten Uraufstellsysteme gemäß Gysi, Gerber und Schreinemaker. An die Aufstelltechniken von Gysi und Gerber anlehnende Aufstelltechniken gemäß APF und APF NT und TiF sind ebenfalls vorteilhaft einsetzbar. Gemäß Staub Cranial wird eine mathematisch berechnende Aufstelltechnik verwendet, die aufgrund von erstellten Messungen die ursprüngliche Position der Zähne bestimmt und dort entsprechend wieder positioniert.

Der anmeldungsgemäße Fräsblock bzw. das Fräsblocksystem ist insbesondere auch bei der Herstellung von Teilprothesen einsetzbar, insbesondere, wenn zumindest gegenüberliegende Quadranten des Ober- bzw. Untergebisses eingesetzt werden können. Aufgrund der für die Zähne in Okklusionsstellung vorbereiteten Aufstellung ist somit auch nach Einsetzen und Einpassen in den entsprechenden Kieferbereich eine Nachbearbeitung der Zähne praktisch nicht notwendig.

Eine besonders vorteilhafte Herstellungstechnik für den Fräsblock bzw. für das Fräsblocksystem ist die Möglichkeit, die ausgewählten Zähne in der Okklusionsstellung in dem ersten Bereich der Prothesenbasis vorpolymerisiert einzusetzen und dann erst bei dem Zusammenfügen mit dem zweiten Bereich der Prothesenbasis die Endpolymerisation vorzunehmen. Auf diese Weise wird erreicht, dass die jeweilige Ober- und Unterkieferprothese einstückig und form- und kraftschlüssig präpariert und hergestellt wird und somit ein geringeres Allergiepotential sowie eine höhere Verbundfestigkeit aufweist.

Weitergehende Ausbildungen der vorliegenden Erfindung sind Gegenstand der weiteren Unteransprüche.

Mit dem anmeldungsgemäßen Fräsblock bzw. Fräsblocksystem wird ermöglicht, dass eine Fixierung bzw. Kennzeichnung jeglicher Art für eine Nulllageübergabe bzw. Nullpunktsübergabe zur maschinellen CAD/CAM Bearbeitung vorgenommen werden kann. Dies ermöglicht eine enorme Zeitersparnis, insbesondere durch Wegfall des Abtastens, zur Erreichung der Lagepositionierung und verhindert Fehlbearbeitungen in einem CAD/CAM System.

Der anmeldungsgemäße Fräsblock sowie das Fräsblocksystem und das Verfahren zur Herstellung von Teil- oder Totalprothesen werden anhand der Zeichnungen nachstehend beispielhaft erläutert.

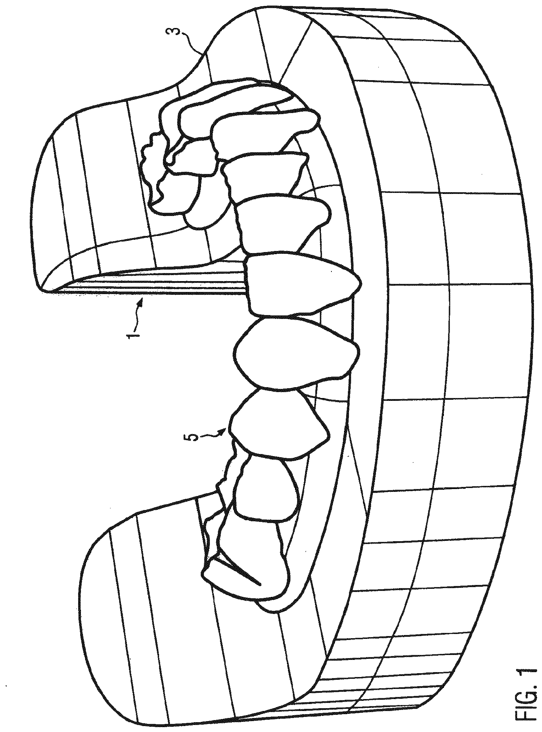

In Figur 1 ist ein Fräsblock 1 dargestellt, der eine Prothesenbasis 3 aufweist, die entsprechend der Kieferform bearbeitet wird, auf der bereits künstlich geformte Zähne 5 eines vollständigen Gebisses vorgesehen sind. Die künstlichen Zähne sind bereits so gearbeitet, dass die Zähne entsprechend einer Okklusionsstellung ausgerichtet sind und somit optimal mit dem nicht dargestellten Oberkiefer zusammenwirken können. In dieser Darstellung sind beispielsweise die Prothesenbasis und die Zähne einstückig ausgebildet, sodass der Herstellungsprozess vereinfacht wurde.

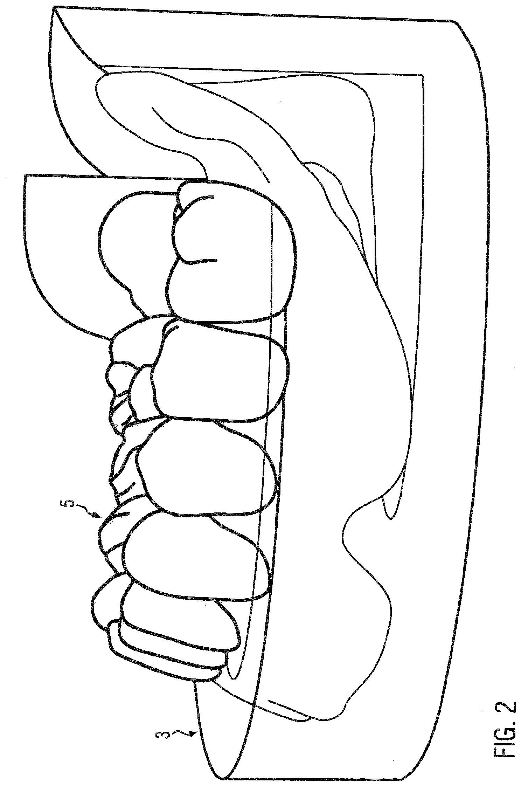

In Figur 2 ist die fertiggestellte Totalprothese des Unterkiefers dargestellt, wobei die Prothesenbasis 3 bereits an die Mundhöhle angepasst ist. Die Figur 2 zeigt deutlich, wie aus der Prothesenbasis die Form der Mundhöhle ausgefräst wird, wobei die Zähne 5, welche in Okklusionsstellung ausgerichtet sind, unbehandelt bleiben. Mit dem anmeldungsgemäßen Verfahren wird somit erreicht, dass lediglich durch Anpassung der Prothesenbasis eine vollständige Totalprothese vorliegt, deren Okklusion stimmig ist und kein weiterer Verfahrensschritt, wie beispielsweise Einpassen in einen Artikulator, erforderlich macht. Auch ein weiterer Verfahrensschritt, wie beispielsweise im Stand der Technik genannt, nämlich die nachträgliche Ausrichtung durch Aufbau der Zähne in Wachs, um die Okklusionsstellung zu erreichen, ist mit dem anmeldungsgemäßen Verfahren nicht erforderlich.

## Patentansprüche

1. Verfahren zur Herstellung von Teil- oder Totalprothesen, welches aus folgenden Schritten besteht:
a. Herstellen bzw. Bereitstellen eines ersten Fräsblocks (1) für das Obergebiss und eines zweiten Fräsblocks für das Untergebiss, wobei der erste und der zweite Fräsblock jeweils eine Prothesenbasis (3) aufweisen, auf der künstlich geformte Zähne (5), vorzugsweise eines vollständigen Gebisses, vorgesehen sind, wobei die Zähne für die Okklusionsstellung aufgestellt sind.
b. Bestimmen der Konturdaten der Mundhöhle eines Patienten;
c. Präparieren und/oder Bearbeiten des ersten und zweiten Fräsblocks, und zwar der Prothesenbasis, entsprechend der Konturdaten der Mundhöhle.

2. Verfahren nach Anspruch 1, wobei das Präparieren und/oder Bearbeiten des ersten und zweiten Fräsblocks mittels CAD/CAM Systemen erfolgt.

3. Verfahren nach einem der Ansprüche 1 bis 2, wobei die Zähne nach einem der Aufstellverfahren Gysi, Gerber, APF, APF NT, TiF, Schreinemaker oder Staub Cranial aufgestellt sind.

4. Verfahren nach einem der Ansprüche 1 bis 3, wobei die ausgewählten künstlich geformten Zähne auf einen zweiten Bereich der Prothesenbasis aufgestellt sind und dieser zweite Bereich mit einem ersten, dem zu bearbeitenden Bereich der Prothesenbasis zusammengefügt ist.

5. Verfahren nach Anspruch 4, wobei der erste und der zweite Bereich durch Polymerisation, durch Kleben, durch Pressen und/oder Verschmelzen zusammengefügt sind.

6. Verfahren nach einem der Ansprüche 1 bis 5, wobei die ausgewählten Zähne in der Okklusionsstellung im zweiten Bereich vorpolymerisiert und/oder anschließend mit dem ersten Bereich endpolymerisiert sind.

7. Verfahren nach einem der Ansprüche 1 bis 6, wobei die Prothesenbasis zur vereinfachten Einpassung in die Mundhöhle bzw. Anpassung an die Kieferform des Patienten flexibel ausgestaltet ist.

8. Verfahren nach Anspruch 7, wobei die flexible Prothesenbasis durch Erwärmen, Bestrahlung mit Licht oder durch chemische Reaktion mindestens zweier in der Prothesenbasis vorhandener Komponenten versteift bzw. verfestigt wird.

9. Verfahren nach einem der Ansprüche 1 bis 8, **dadurch gekennzeichnet, dass** die palatinalen und vestibulären Bereiche der Prothesenbasis nicht bearbeitet werden.

10. Verfahren nach einen der Ansprüche 1 bis 9, **dadurch gekennzeichnet, dass** das Bereitstellen des ersten Fräsblocks für das Obergebiss und des zweiten Fräsblocks für das Untergebiss durch Auswahl aus einer Gruppe von mindestens 3 unterschiedlichen Fräsblocksets mit mindestens 3 unterschiedlichen Zahnreihen erfolgt.

11. Verfahren nach Anspruch 10, **dadurch gekennzeichnet, dass** die Zahnreihen aus 14 Zähnen jeweils für das Ober- und Untergebiss bestehen.

## Claims

1. Method for producing partial or total prostheses, which method consists of the following steps:
a. producing or making available a first milling block (1) for the upper set of teeth and a second milling block for the lower set of teeth, wherein the first and second milling block each have a prosthesis base (3) on which synthetically moulded teeth (5), preferably of a full set of teeth, are provided, wherein the teeth are set up for the occlusion position;
b. determining the contour data of the oral cavity of a patient;
c. preparing and/or processing the first and second milling block, specifically the prosthesis base, according to the contour data of the oral cavity.

2. Method according to Claim 1, wherein the preparation and/or processing of the first and second milling block takes place by means of CAD/CAM systems.

3. Method according to either of Claims 1 and 2, wherein the teeth are set up according to one of the Gysi, Gerber, APF, APF NT, TiF, Schreinemaker or Staub Cranial set-up methods.

4. Method according to one of Claims 1 to 3, wherein the selected synthetically moulded teeth are set up on a second region of the prosthesis base, and this second region is joined to a first region of the prosthesis base, the region to be processed.

5. Method according to Claim 4, wherein the first and second region are joined together by polymerization, by adhesive bonding, by pressing together and/or melting together.

6. Method according to one of Claims 1 to 5, wherein the selected teeth are pre-polymerized in the occlusion position in the second region and/or then finally polymerized to the first region.

7. Method according to one of Claims 1 to 6, wherein the prosthesis base is flexible in order to permit simplified fitting into the oral cavity and/or adaptation to the shape of the jaw of the patient.

8. Method according to Claim 7, wherein the flexible prosthesis base is stiffened or solidified by heating, by irradiating with light, or by chemical reaction of at least two components present in the prosthesis base.

9. Method according to one of Claims 1 to 8, **characterized in that** the palatal and vestibular regions of the prosthesis base are not processed.

10. Method according to one of Claims 1 to 9, **characterized in that** the first milling block for the upper set of teeth and the second milling block for the lower set of teeth are made available by selection from a group of at least 3 different milling block sets having at least 3 different rows of teeth.

11. Method according to Claim 10, **characterized in that** the rows of teeth are each composed of 14 teeth, each for the upper set of teeth and the lower set of teeth.

## Revendications

1. Procédé de fabrication de prothèses partielles ou totales, qui comprend les étapes suivantes :
a. fabriquer ou fournir un premier bloc à fraiser (1) destiné à la denture supérieure et un deuxième bloc à fraiser destiné à la denture inférieure, le premier et le deuxième bloc à fraiser comportant chacun une base de prothèse (3) sur laquelle sont prévues des dents artificielles (5), de préférence une denture complète, les dents étant placées pour la position d'occlusion.
b. déterminer les données de contour de la cavité buccale d'un patient ;
c. préparer et/ou traiter des premier et deuxième blocs à fraiser, c'est-à-dire la base de prothèse, conformément aux données de contour de la cavité buccale.

2. Procédé selon la revendication 1, la préparation et/ou le traitement des premier et deuxième blocs à fraiser étant effectués à l'aide de systèmes CAD/CAM.

3. Procédé selon l'une des revendications 1 à 2, les dents étant placées selon l'un des procédés de mise en place Gysi, Gerber, APF, APF NT, TiF, Schreinemaker ou Staub Cranial.

4. Procédé selon l'une des revendications 1 à 3, les dents formées artificiellement qui ont été sélectionnées étant mises en place sur une deuxième zone de la base de prothèse et cette deuxième zone étant assemblée avec une première zone de la base de prothèse à traiter.

5. Procédé selon la revendication 4, la première et la deuxième zone étant assemblées par polymérisation, par collage, par pressage et/ou fusion.

6. Procédé selon l'une des revendications 1 à 5, les dents sélectionnées étant pré-polymérisées dans la position d'occlusion dans la deuxième zone et/ou soumises ensuite à une polymérisation finale avec la première zone.

7. Procédé selon l'une des revendications 1 à 6, la base de prothèse étant conçue pour être souple afin de simplifier l'enchâssement dans la cavité buccale ou l'adaptation à la forme de la mâchoire du patient.

8. Procédé selon la revendication 7, la base de prothèse souple étant rigidifiée ou solidifiée par chauffage, irradiation à la lumière ou réaction chimique d'au moins deux composants présents dans la base de prothèse.

9. Procédé selon l'une des revendications 1 à 8, **caractérisé en ce que** les zones palatales et vestibulaires de la base de prothèse ne sont pas traitées.

10. Procédé selon la revendication 1, **caractérisé en ce que** le premier bloc à fraiser destiné à la denture supérieure et le deuxième bloc à fraiser destiné à la denture inférieure sont fournis par sélection dans un groupe comprenant au moins 3 ensembles de blocs à fraiser différents comportant au moins 3 rangées de dents différentes.

11. Procédé selon la revendication 10, **caractérisé en ce que** les rangées de dents comprennent 14 dents chacune pour la denture supérieure et la denture inférieure.
